# EUROPEAN PATENT APPLICATION

(11) **EP 1 975 169 A1**
(43) Date of publication of application: **01.10.2008**
(21) Application number: 07117552.5
(22) Date of filing: 28.09.2007
(51) Int. Cl.: C07D 417/14

(54) **Process for the preparation of ziprasidone**

(71) Applicant: INKE, S.A., 08755 Castellbisbal (ES)
(72) Inventor: Huguet Clotet, Joan, E-08970, Sant Joan Despí - Barcelona (ES); Puig Torres, Salvador, E-08005, Barcelona (ES)
(74) Representative: ABG Patentes, S.L.

(57) **Abstract**

The present invention relates to an improved method for the preparation of ziprasidone comprising the steps of mixing 5-(2-chloroethyl)-6-chloro-1,3-dihydroindole-2(2H)-one with either a free base or salt form of 3-(1-piperazinyl)-1,2-benzoisothiazole in the presence of a base and an organic solvent characterized in that the organic solvent is a polar aprotic solvent or a mixture of polar aprotic solvents and that the reaction requires the addition of 0,1-0,8 Equivalents of an halide salt, preferably 0,3-0,6 Equivalents, more preferably 0,4-0,5 Equivalents.

## Description

### Field of the invention

The present invention relates to processes for the preparation of ziprasidone.

### Background of the invention

Ziprasidone (I) is an antipsychotic agent that is chemically unrelated to phenothiazine or butyropheneone antipsychotic agents. Ziprasidone has the following chemical name 5-[2-[4-(1,2-benzisothiazol-3-yl)-1-piperazinyl]ethyl]-6-chloro-1,3-dihydro-2H-indol-2-one and structure:

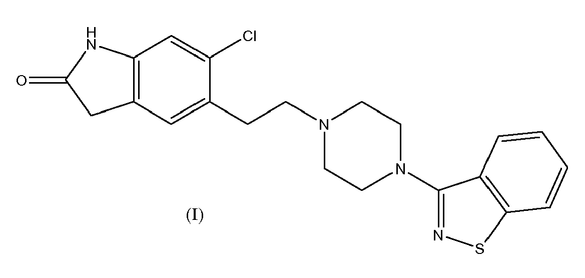

Ziprasidone is administered for the treatment of psychiatric disorders such as schizophrenia. Other uses of ziprasidone are disclosed in WO 03/070246 and WO 01/91756 incorporated herein by reference. A process for the preparation of ziprasidone hydrochloride monohydrate having a mean particle size equal to or less than about 85 µm is also disclosed in European patent application EP 0 965 343 A.

Ziprasidone is marketed under the name GEODON as an oral capsule and as an injectable drug. GEODON capsules contain the monohydrate hydrochloride salt of ziprasidone, and come in 20, 40, 60 and 80 mg dosage forms. GEODON for injection contains ziprasidone mesylate trihydrate and contains 20 mg base equivalent of ziprasidone.

European patent EP 0 281 309 B1 discloses a procedure for the preparation of ziprasidone. Example 16, ziprasidone hydrochloride hemihydrate is prepared by reaction between 1,2-benzisothiazole-3-piperazinyl ("BITP") and 5-(2-chloroethyl)-6-chloro-1,3-dihydroindole-2(2H)-one ("CEI") in methyl-iso-butyl ketone with sodium carbonate as base and in the presence of a catalytic amount of sodium iodide. The provided yield for this process is 20%.

European patent EP 0 584 903 B1 discloses a process for the preparation of ziprasidone hydrochloride. This process consists of reacting BITP with CEI in water and in the absence of organic solvents. In Example 1 of this patent, a solution of sodium carbonate in water is mixed with CEI and BIPT. The mixture is then stirred and heated to reflux for about 9 to 14 hours, slurried to room temperature for 1 hour and then filtered. The obtained free base is converted into the hydrochloride salt which is isolated in 86% weight yield.

In European patent application EP 0 586 191 A a solution of sodium carbonate in water is mixed with BITP and CEI, and the resulting slurry is heated at reflux for 14 hours. The reaction mixture is then cooled and filtered. The wet product is then re-slurried in isopropyl alcohol at room temperature for 2 hours.

WO 2005/040160 discloses an improved process for the preparation of ziprasidone comprising the steps of reacting BIPT with CEI in water in the presence of a base and a non-basic ionic additive.

WO 2006/047893 describes a process for the preparation of ziprasidone which comprises mixing BIPT and CEI with an alkaline compound in an organic solvent or a mixture of organic solvents, heating at reflux temperature until the reaction is complete, cooling and adding this mixture to water. According to WO 2006/047893, organic solvents useful in the cited reaction include poly(ethyleneglycol), poly(ethylene glycol)methyl ether cyclic or acyclic amides such as 1-methyl-2-pyrrolidinone; dialkyl sulfones and their mixtures.

In some cases, the ziprasidone obtained in those procedures was purified by column chromatography, thus making the process impractical for large scale preparations. Other processes use potentially explosive gases such as hydrogen. Other processes use very large volumes of solvents such as tetrahydrofuran to accomplish the purification of crude ziprasidone. Finally, other processes take a large number of hours to obtain the desired product.

The present invention provides a rapid process for the preparation of ziprasidone in high yields and purity, suitable for large-scale manufacturing, which helps to overcome the deficiencies of the prior art.

### SUMMARY OF THE INVENTION

Inventors have surprisingly found that the use of a polar aprotic solvent and the addition of 0.1-0.8 Equivalents of an halide salt in the condensation reaction between 5-(2-chloroethyl)-6-chloro-1,3-dihydroindole-2(2H)-one ("CEI") with either a free base or salt form of 1,2-benzisothiazole-3-piperazinyl ("BITP") allows a fast obtaining of ziprasidone in high yields and purity.

Thus, an aspect of the present invention is to provide an improved process for the preparation of ziprasidone comprising the steps of:
(a) mixing 5-(2-chloroethyl)-6-chloro-1,3-dihydroindole-2(2H)-one with either a free base or salt form of 1,2-benzisothiazole-3-piperazinyl in the presence of a base and an organic solvent,
(b) heating the mixture obtained in step (a) and stirring for a sufficient amount of time to obtain ziprasidone formation,
(c) cooling the mixture obtained in step (b) and adding water, and
(d) isolating crude ziprasidone;
characterized in that the organic solvent is a polar aprotic solvent or a mixture of polar aprotic solvents and that the reaction requires the addition in step (a) of 0,1-0,8 Equivalents of an halide salt, preferably 0,3-0,6 Equivalents, more preferably 0,4-0,5 Equivalents.

### DESCRIPTION OF THE INVENTION

According to an aspect of the present invention, it is provided an improved method for the preparation of ziprasidone comprising the steps of:
(a) mixing 5-(2-chloroethyl)-6-chloro-1,3-dihydroindole-2(2H)-one with either a free base or salt form of 1,2-benzisothiazole-3-piperazinyl in the presence of a base and an organic solvent,
(b) heating the mixture obtained in step (a) and stirring for a sufficient amount of time to obtain ziprasidone formation,
(c) cooling the mixture obtained in step (b) and adding water, and
(d) isolating crude ziprasidone;
characterized in that the organic solvent is a polar aprotic solvent or a mixture of polar aprotic solvents and that the reaction requires the addition in step (a) of 0,1-0,8 Equivalents of an halide salt, preferably 0,3-0,6 Equivalents, more preferably 0,4-0,5 Equivalents.

The inventors have discovered that the use of aprotic solvents and the addition in step (a) of 0.1-0.8 Equivalents of an halide salt accelerates the condensation reaction which is complete in approximately 8 hours and provides high yields of ziprasidone without detectable impurities.

According to a preferred embodiment of the invention, the polar aprotic solvent used in the process is selected from the group consisting of dimethylacetamide, tetramethylurea, 1,3-dimethyl-2-imidazolidinone and 4,4-dimethyl-2-imidazolidinone. More preferably, the solvent used is dimethylacetamide.

According to a preferred embodiment the free base is selected from sodium carbonate, potassium carbonate, lithium carbonate, sodium bicarbonate, potassium bicarbonate and the like, more preferably sodium carbonate.

Preferably, the halide salt is selected from NaI, KI, KBr and NaBr. More preferably, the halide salt is NaBr.

In a particular embodiment, the temperature of the reaction in step (b) is not more than 100°C.

According to another embodiment of the invention, crude ziprasidone obtained trough the above cited process is purified. The purification of crude ziprasidone comprises:
(a) suspending crude ziprasidone in acetone or tetrahydrofuran at reflux temperature, and
(b) cooling the mixture obtained in step (a), filtering, washing with acetone or tetrahydrofuran and isopropyl alcohol and isolating the product

The ziprasidone obtained according to the teachings of the present invention can be converted into differents salts, such as the hydrochloride and the mesylate salts.

The following examples are displayed to illustrate the aspects of the present invention. They do not intend to limit in any way the scope of the invention defined in the present description

### EXAMPLES

### Example 1

To 128 mL of a stirred solution of 32g of 5-(2-chloroethyl)-6-chloro-1,3-dihydroindole-2(2H)-one and 42,4g of 1,2-benzisothiazole-3-piperazinyl hydrochloride in dimehtylacetamide are added at room temperature, 14,68g of Na₂CO₃ and 6,4g of NaBr. The resulting solution is stirred at 95-100°C during 8 hours. Then, the solution is cooled until 70°C approximately and 200 mL of water are added while stirring. The suspended solid is filtered, washed with water and isopropyl alcohol. Once dried, 51,4g of crude ziprasidone is obtained (90% yield).
1H-RMN (250MHz, DMSO), ppm: 10,42 (1H, s); 8,06 (2H, d, J=8,85Hz); 7,56 (1H, t, J=7,60Hz); 7,43 (1H, t, J=8,25Hz); 7,24 (1H, s); 6,81 (1H, s), 3,46 (4H, m); 3,33 (2H, s); 2,85 (2H, m); 2,70 (4H, m); 2,56 (2H, m).
IR (cm-1):2806 (-CH, d), 2750 (-CH, d), 1727 (CO, i), 1663 (d), 1628 (m), 1484 (i), 1450 (NH, m), 1384 (i), 1320 (CN, i), 1250 (i), 1135 (i), 1001 (i), 736 (i), 679 (m).

### Example 2

A solution of 2.6g of crude ziprasidone of Example 1 in 10.3 ml of acetone is heated at reflux during 30 min. After that time, the solution is cooled to 30-35°C and the solid is filtered and washed with acetone. 2.5 g pure Ziprasidone are obtained.

### Example 3

A solution of 2.1g of crude ziprasidone of Example 1 in 4.3 mL of tetrahydrofuran is heated at reflux during 30 min. After that time, the solution is cooled to 65-67°C and the solid is filtered and washed with tetrahydrofuran and IPA (isopropyl alcohol). 1.5 g pure Ziprasidone are obtained. The physical properties of the product obtained corresponds to the product of example 3 of EP 1 029 861 B1.

### Example 4: Assays of crystallization of ziprasidone

Ziprasidone obtained following Example 2 and 3 was crystallized in different experimental conditions as shown in the following table:

| Assay | Experimental conditions |
|---|---|
| 1 | Preparing a dissolution of 2g of ziprasidone in 13,2 mL of dimethylacetamide. Heating at 110°C during 15 min. Adding 4,5 mL water while cooling. Filtering the precipitate and washing with isopropanol Yield: 87% |
| 2 | Preparing a solution of 1g g or crude ziprasidone in a mixture of 24,5 mL of tetrahydrofuran and 2 mL of water. Heating at reflux temperature about 25-35 min. Filtering the precipitate and washing with isopropanol Yield: 81% |
| 3 | Preparing a solution of 1g of crude ziprasidone in 40 mL tetrahydrofuran and heating at reflux during 1h. Filtering the precipitate and washing with isopropanol Yield: 62% |

### Example 5: Preparation of ziprasidone·HCl

Crystalline ziprasidone base obtained following one of the above cited assays was converted into its hydrochloride salt according to one of the procedures described below:

| Assay | Experimental conditions |
|---|---|
| 1 | Dissolving 5g of ziprasidone base in 15 mL formic acid. Heating at 20°C. Adding 15 mL methanol and 15 mL water at this temperature and stirring 10 min. Filtering and adding 11 mL HCl 15%. Stirring during 30 min at room temperature. Filtering the solid product and washing with tetrahydrofuran. Yield: 90% |
| 2 | Dissolving 1.8g of ziprasidone base in 4.5 mL formic acid. Heating at 25-30°C. When the solid is dissolved, adding 4.5 mL tetrahydrofuran and 2 mL water. Stirring 30 min at 25-30°C. Filtering and washing with 1 mL formic acid/THF/water 1:1:1. Stirring and adding 2.5 mL water. Slowly adding 3.3 ml HCl 15% during 20 min. Stirring during 30 min at room temperature. Filtering the solid product and washing with tetrahydrofuran. Yield: 90% |
| 3 | Dissolving 1g ziprasidone base in a mixture of 26 ml tetrahydrofuran and 3 mL water. Heating at 64°C during 30 min, filtering and rinsing with tetrahydrofuran. Slowly adding 2 mL water and 0.29 mL concentrated HCl during 30 min and stirring at room temperature over 2 hours. Then, stirring the reaction mixture at 1-5°C during 10 hours. Filtering the solid and washing with cold tetrahydrofuran. Yield: 92% |
| 4 | 3g ziprasidone mesylate prepared according Example 4 of EP 904 273 B1 are dissolved in 27 mL of formic acid/THF/water 1:1:1 at room temperature. Slowly adding 6 mL of HCl 15% and stirring 1h at room temperature. Filtering and washing the resulting solid with THF. Yield: 98% |

Ziprasidone hydrochloride obtained through the above assays corresponds to Ziprasidone hydrochloride monohydrate described and characterized in US patent No. 5,312,925.

## Claims

1. A process for the preparation of ziprasidone comprising the steps of:
(a) mixing 5-(2-chloroethyl)-6-chloro-1,3-dihydroindole-2(2H)-one with either a free base or salt form of 1,2-benzisothiazole-3-piperazinyl in the presence of a base and an organic solvent,
(b) heating the mixture obtained in step (a) and stirring for a sufficient amount of time to obtain ziprasidone formation,
(c) cooling the mixture obtained in step (b) and adding water, and
(d) isolating crude ziprasidone;
**characterized in that** the organic solvent is a polar aprotic solvent or a mixture of polar aprotic solvents and that the reaction requires the addition in step (a) of 0,1-0,8 Equivalents of an halide salt, preferably 0,3-0,6 Equivalents, more preferably 0,4-0,5 Equivalents.

2. The process according to claim 1 wherein the polar aprotic solvent is selected from dimethylacetamide, tetramethylurea, 1,3-dimethyl-2-imidazolidinone and 4,4-dimethyl-2-imidazolidinone.

3. The process according to claim 2 wherein the the polar aprotic solvent is dimethylacetamide.

4. The process according to claim 1 wherein the free base is selected from sodium carbonate, potassium carbonate, lithium carbonate, sodium bicarbonate and potassium bicarbonate.

5. The process of claim 4 wherein the free base is sodium carbonate.

6. The process according to claim 1 wherein the halide salt is selected from NaI, KI, KBr and NaBr.

7. The process according to claim 6 wherein the halide salt is NaBr.

8. The process according to claim 1 wherein the temperature of the reaction in step (b) is not more than 100°C.

9. The process of claim 1 further comprising the purification of crude ziprasidone.

10. The process of claim 9 wherein the purification of crude ziprasidone comprises:
(a) suspending crude ziprasidone in acetone or tetrahydrofuran at reflux temperature, and
(b) cooling the mixture obtained in step (a), filtering, washing with acetone or tetrahydrofuran and isopropyl alcohol and isolating the product
